# EUROPEAN PATENT APPLICATION

(11) **EP 2 319 408 A1**
(43) Date of publication of application: **11.05.2011**
(21) Application number: 09173132.3
(22) Date of filing: 15.10.2009
(51) Int. Cl.: A61B 5/022, A61B 5/0225, A61B 5/024

(54) **Device for controlling the pressure in an inflatable pressure pad**

(71) Applicant: Finapres Medical Systems B.V., 7575 EJ Oldenzaal (NL)
(72) Inventor: Rutgers, Petrus Theodorus, 7556 BW, Hengelo (NL); Leideman, Jan, 7595 AE, Weerselo (NL); Hakvoort, Wouter Bernardus Johannes, 7522 DG, Enschede (NL); Langewouters, Gerardus Johannes, 2421 TT Nieuwkoop (NL)
(74) Representative: 't Jong, Bastiaan Jacob

(57) **Abstract**

The invention relates to a device for controlling the pressure in a finger cuff, the device comprising:
- a first chamber in fluid connection with a finger cuff;
- a movable wall part defining at least a part of a wall of the first chamber;
- an air supply for supplying air to the first chamber and the finger cuff; and
- a pressure sensor for registering the pressure in the first chamber.

The invention also relates to blood pressure measuring device.

## Description

The invention relates to a device for controlling the pressure in an inflatable pressure pad, such as a finger cuff. Such devices are known from for example WO 00/59369.

A device according to the preamble is typically used to measure the blood pressure in a non invasive way by performing plethysmography. To this end a finger cuff is arranged around a finger of a patient. The finger cuff comprises an infrared light source, an infrared sensor and an inflatable bladder. The infrared light is sent through the finger in which a finger artery is present. The infrared sensor picks up the infrared light. The amount of infrared light registered by the sensor is inversely proportional to the artery diameter and indicative for the pressure in the artery.

By inflating the bladder present in the finger cuff, a pressure is exerted on the finger artery. If the pressure is high enough, it will compress the artery and the amount of light registered by the sensor will increase. The amount of pressure necessary in the inflatable bladder to compress the artery is dependent on the blood pressure.

Now by controlling the pressure in the inflatable bladder such that the diameter of the finger artery is kept constant, the blood pressure can be monitored in very good detail as the pressure in the inflatable bladder is directly linked to the blood pressure.

The device for controlling the pressure in a finger cuff according to the prior art uses a pump, which is constantly supplying air, or at least at each moment the pressure needs to rise in the finger cuff. An inlet valve and an outlet valve are provided to supply the inflatable bladder of the finger cuff with the air from the pump to increase the pressure and to discharge air to decrease the pressure. As the blood pressure rises and drops with each heart beat, the pump needs to pump constantly and air is constantly discharged. A buffer can be provided to relieve the pump.

The device according to the prior art has the disadvantage of a high energy consumption as a result of the continuous operating pump and the disadvantage of noise due to the pump and the regular discharge of air. This is undesired in particular for applications such as ambulatory use,application in a quiet examination room and while performing measurements during sleep.

It is an object of the invention to reduce or even prevent the above mentioned disadvantages.

This object is achieved with a device according to the invention, which device comprises:
- a first chamber in fluid connection with an inflatable pressure pad;
- a movable wall part defining at least a part of a wall of the first chamber;
- an air supply for supplying air to the first chamber and the inflatable pressure pad; and
- a pressure sensor for registering the pressure in the first chamber.

With the device according to the invention, the blood pressure profile can be followed more efficiently, as the high frequency part of the profile is provided with the movable wall part. Furthermore, it is no longer necessary to have a pump constantly operating to supply air, or to discharge air as soon as a decrease of pressure is desired in the inflatable pressure pad.

With the device according to the invention, the first chamber and the connected inflatable pressure pad, like a finger cuff, are supplied with air up to a pressure substantially equal to the mean blood pressure. Any desired fluctuations around the mean blood pressure to follow the actual blood pressure due to for example the heart rhythm, are provided by moving the movable wall part causing an increase or decrease of the first chamber volume and accordingly a decrease or increase respectively of the pressure in the chamber. So, with the device according to the invention, it is no longer necessary to supply or discharge air to follow the fluctuation in blood pressure. However, it could be desired to control the air supply to assist in following a low frequency component of the blood pressure profile, for example when some leakage occurs. The movable wall will at least always be used for following the high frequency part of the pressure profile.

An embodiment of the device according to the invention comprises an actuator for moving the movable wall part. This actuator is preferably a electromagnet, but could also be a motor with a mechanical transmission or a piezo element with a suitable transmission.

A preferred embodiment of the device according to the invention comprises a second chamber, wherein the movable wall part is at least part of a wall of the second chamber. With the second chamber opposite of the movable wall it is possible to provide some counterforce to the movable wall, such that only energy is put in moving the wall to cause pressure differences.

The counterforce could be generated by a spring, but preferably the air supply is also connected to the second chamber to supply the second chamber with air. When the pressure is raised in both the first chamber and second chamber to the level of the mean blood pressure, only energy is needed for moving the wall to cause variation of pressure relative to the mean pressure.

In yet another embodiment, the device according to the invention comprises a bypass channel arranged between the first and second chamber, wherein the air supply is directly connected to either one of the first chamber and the second chamber and wherein the bypass channel has a restriction.

With the bypass channel, the pressure between the two chambers will be equalized. To be able to apply quick pressure differences in the first chamber by moving the movable wall part, a restriction is arranged in the bypass channel. This provides a delay in the equalization of the pressure in both chambers, allowing for short pressure differences in the first chamber.

The restriction should be chosen such that the delay is long enough to provide pressure differences necessary to follow the pressure fluctuations in the blood pressure relative to the mean blood pressure.

The restriction could be made variable, such that both chambers could be brought quickly to the same pressure.

In a preferred embodiment of the device according to the invention the movable wall part is a movable membrane. A movable membrane provides for simple solution, while ensuring air tightness of the movable wall part.

In another embodiment of the device according to the invention the movable wall part is a plunger. The clearance of the moving parts of the plunger could be chosen such that a bypass channel is provided for in the plunger itself.

The invention further relates to a blood pressure measuring device, comprising:
- an inflatable pressure pad, such as a finger cuff having an infrared light source, an infrared sensor and an inflatable bladder for performing plethysmography;
- a device for controlling the pressure in an inflatable pressure pad according to the invention, wherein the first chamber of the device is in fluid connection with the inflatable bladder of the inflatable pressure pad;
- control means for controlling the pressure in the inflatable bladder by supplying air to the first chamber and by moving the movable wall part in order to maintain a constant signal of the infrared sensor.

These and other advantages and features of the invention will be elucidated in conjunction with the accompanying drawings. The control means should control the cuff pressure taking the limited stroke of the movable wall into account. In a preferred configuration this is realized by a control means that actuates the movable wall to provide the high-frequency components of the cuff pressure and the air-supply to provide the low-frequency components of the cuff-pressure.
Figure 1 shows a hand with a finger cuff and a device for measuring the blood pressure.
Figure 2 shows schematically an embodiment of a device for controlling the pressure in a finger cuff according to the invention.
Figure 3 shows a diagram of the blood pressure of a patient.

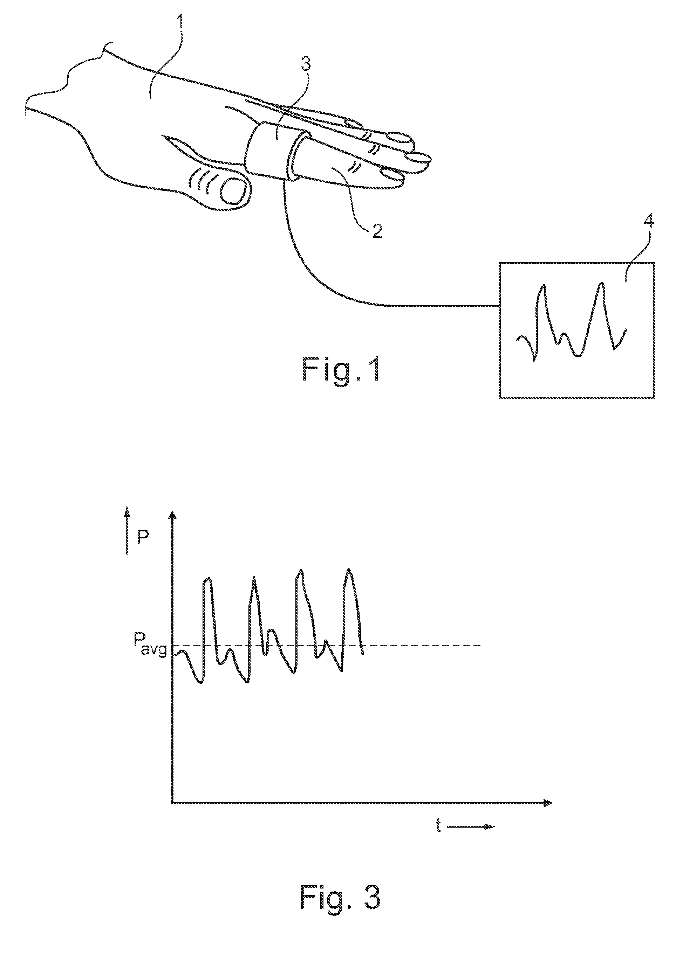

In figure 1 a hand 1 is shown, which carries around the index finger 2 a finger cuff 3. The finger cuff 3 is connected to a blood pressure measuring device 4.

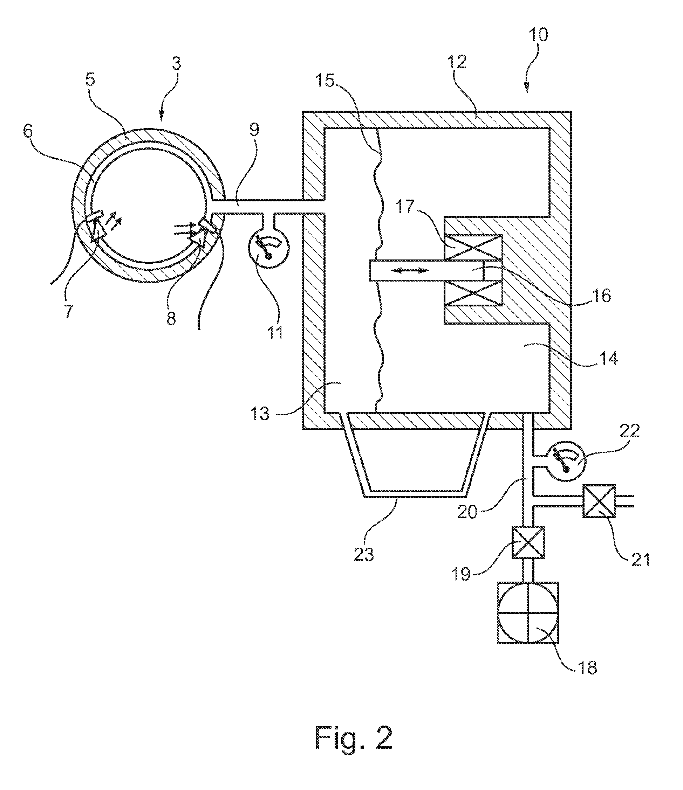

Figure 2 shows a schematic view of an embodiment of a device for controlling the pressure in the finger cuff 3. The finger cuff 3 has a rigid or semi-rigid outer sleeve 5 and an inflatable bladder 6 arranged in the sleeve 5. The cuff 3 furthermore comprises an infrared LED 7 and an infrared sensor 8. The amount of infrared light going through a finger 2 and being registered by the sensor 8 is inversely proportional to the diameter of the artery of the finger 2. The diameter of the artery is in turn indicative for the blood pressure in the artery

The inflatable bladder 6 is connected through a line 9 with a device 10 for controlling the pressure in the bladder 6. A pressure sensor 11 is arranged in the line 9 to feed the measurements to a controller.

The device 10 has a housing 12 with a first chamber 13 and a second chamber 14. The housing 12 is divided into the two chambers 13, 14 by the membrane 15. This membrane is movable by an actuator having a rod 16, which is operated by a coil 17.

By moving the membrane 16 the volume of the first chamber 13 can be changed and accordingly the pressure in the chamber 13 can be altered.

The device 10 further has an air pump 18. This pump 18 is connected via a valve 19 and a line 20 to the second chamber 14. The line 20 also comprises an exhaust valve 21 and a pressure sensor 22.

A bypass channel 23 is provided between the first chamber 13 and the second chamber 14. This bypass channel 23 is designed such that it restricts the air flow between the two chambers or a separate additional restriction (not shown) is added to this channel 23 to provide the desired restriction.

In operating of the device 10, the pump 18 is energized and valve 19 is opened such that air is pumped into the second chamber 14. Via the bypass channel 23 also the first chamber 13 is provided with air. As soon as the pressure measured by the sensor 11 reaches the average blood pressure P_{avg} the valve 19 is closed. The blood pressure of a patient is schematically shown in figure 3. The valve 19 can be discarded off when the pump 18 can be started and stopped quick enough without causing high pressure disturbances in the system.

The control means of the blood pressure measuring device then measure the diameter of the artery by the infrared sensor 8. If the diameter decreases, the pressure in the bladder 6 and first chamber has to be decreased, while with an increase of the diameter the pressure has to be raised. In order to achieve these pressure fluctuations relative to the average blood pressure P_{avg}, the membrane 15 is moved by the actuator 16, 17. By decreasing the volume of the first chamber 13, the pressure is increased, while increasing the volume of the first chamber 13 results in a decrease of the pressure.

As a result, the required average pressure and fluctuation of the pressure for the finger cuff can be achieved with the invention, without having to constantly supply air from the pump 18 or the air discharge. This reduces the energy consumption and reduces the generated noise.

## Claims

1. Device for controlling the pressure in an inflatable pressure pad, such as a finger cuff, the device comprising:
- a first chamber in fluid connection with an inflatable pressure pad;
- a movable wall part defining at least a part of a wall of the first chamber;
- an air supply for supplying air to the first chamber and the inflatable pressure pad; and
- a pressure sensor for registering the pressure in the first chamber.

2. Device according to claim 1, comprising an actuator for moving the movable wall part.

3. Device according to claim 1 or 2, comprising a second chamber, wherein the movable wall part is at least part of a wall of the second chamber.

4. Device according to claim 3, wherein the air supply is also connected to the second chamber to supply the second chamber with air.

5. Device according to claim 3, comprising a bypass channel arranged between the first and second chamber, and wherein the air supply is directly connected to one of the first chamber and the second chamber and wherein the bypass channel has a restriction.

6. Device according to any of the preceding claims, wherein the movable wall part is a movable membrane.

7. Device according to any of the claims 1 - 6, wherein the movable wall part is a plunger.

8. Blood pressure measuring device, comprising:
- an inflatable pressure pad, such as a finger cuff, having an infrared light source, an infrared sensor and an inflatable bladder for performing plethysmography;
- a device according to any of the preceding claims, wherein the first chamber of the device is in fluid connection with the inflatable bladder of the inflatable pressure pad;
- control means for controlling the pressure in the inflatable bladder by supplying air to the first chamber and by moving the movable wall part in order to maintain a constant signal of the infrared sensor.
